# EUROPEAN PATENT SPECIFICATION

(11) **EP 2 432 864 B1**
(45) Date of publication and mention of the grant of the patent: **14.11.2018**
(21) Application number: 10722995.7
(22) Date of filing: 17.05.2010
(51) Int. Cl.: C12H 1/14, C07K 14/39, C12P 21/00, C12P 21/06

(54) **PROCESS TO PRODUCE A WINE OR FRUIT JUICE STABILISER**
VERFAHREN ZUR HERSTELLUNG EINES WEINSTABILISATORS
PROCÉDÉ DE FABRICATION D'UN STABILISATEUR DE VIN

(30) Priority: 18.05.2009 EP 09160499
(43) Date of publication of application: 28.03.2012
(73) Proprietor: Rymco International AG, 6301 Zug (CH)
(72) Inventor: LANKHORST, Peter Philip, NL-3051 JE Rotterdam (NL)
(74) Representative: Curran, Clair
(86) International application number: PCT/EP2010/056726
(87) International publication number: WO 2010/133543

(56) References cited:
- WO-A1-03/104382
- WO-A1-2006/067145
- WO-A1-2006/067147
- WO-A1-2008/128972
- WO-A2-02/45524
- US-A- 6 139 891
- DUPIN I V S ET AL: "Saccharomyces cerevisiae Mannoproteins That Protect Wine from Protein Haze: Their Release during Fermentation and Lees Contact and a Proposal for Their Mechanism of Action", JOURNAL OF AGRICULTURAL AND FOOD CHEMISTRY, AMERICAN CHEMICAL SOCIETY, WASHINGTON, US, vol. 48, no. 8, 1 January 2000 (2000-01-01), pages 3098-3105, XP002326103, ISSN: 0021-8561
- COMUZZO P ET AL: "Technological application of a yeast industrial derivative to tartaric and protein stabilization of white wines", SCIENCES DES ALIMENTS, LAVOISIER ABONNEMENTS, PARIS, FR, vol. 24, no. 5, 1 January 2004 (2004-01-01), pages 371-382, XP009115450, ISSN: 0240-8813

## Description

### Field of the invention

The present invention relates to a process to produce a wine stabiliser comprising contacting a mannoprotein with a prolyl-specific endoprotease.

### Background of the invention

Tartaric acid is the main organic acid produced by the grape berry during its development. In wine making, during fermentation of the must the solubility of salts of tartaric acid decreases with the increase of ethanol concentration. In young wines, potassium hydrogen tartrate (KHT) is always present in supersaturated concentrations and crystallises spontaneously. After bottling wine the KHT-instability may become a problem due to the unpredictable character of the crystallisation: consumers often perceive the presence of crystals in the bottle as a sign of inferior quality of the wine. Physical treatments can be used prior to bottling of the wine to prevent crystallisation of tartrate salts. These treatments consist of promoting crystallisation by cooling the wine to -4°C or of eliminating the potassium and tartaric ions by electrodialysis or by using ion- exchange resins. However, these time- and energy-consuming processes are supposed to alter the colloidal equilibrium of wine.

The alternative to such physical treatments of wine is to use wine stabilisers preventing the nucleation and/or the growth of crystals of KHT crystals. WO2006/067145 describes a mannoprotein which is fully soluble in wine which can be used as a wine stabiliser. WO2008/128972 describes a wine stabiliser composition comprising at least 2.5% of a peptide mixture with a molecular weight between 3 kDa and 10 kDa.

However, wine stabilisers, particularly wine stabilisers comprising proteins and/or peptides, may have the disadvantage that, once added to the wine, they may react with polyphenols present in said wine resulting in undesirable turbidity. Such turbidity is sometimes referred to as protein haze. Polyphenols are compounds having a chemical structure containing at least two aromatic rings substituted with at least one hydroxyl group or having a chemical structure containing at least one aromatic ring substituted with at least two hydroxyl groups. Examples of such polyphenols are tannins and flavonoids, which include for example catechins, flavonols and anthocyanins. Tannins are defined herein according the International Oenological Codex (Oenological tannins, E-COEI-1-TANNIN 1 , Oenological Tannins INS N°: 181) as a mixture of glucosides either from gallic acid (gallotannins) or from dilactone, ellagic acid (ellagitannin, hydrolysable tannins) or from a mixture of proanthocyanidines (condensed tannins). A wine with protein haze is not appreciated by the customer and may be interpreted as a sign of inferior quality of the wine. Furthermore, if a proteinaceous stabiliser reacts with polyphenols in the wine to form a protein haze, said stabiliser is no longer in solution, which reduces the efficacy of the stabiliser against crystallisation of KHT.

There is a need for wine stabiliser, particularly a manno-protein based stabiliser for wine that can prevent or retard the crystallisation of KHT without causing protein haze.

### Detailed description of the invention

The invention provides a process to produce a wine stabiliser as defined in claim 1.

"Proteinaceous material" is defined herein as any material comprising amino acids, peptides, or proteins, or a mixture thereof. A peptide is defined herein as a compound comprising two or more amino acids which are linked through peptide bonds. In this text, the words peptide and protein are used interchangeably. The amino acids, peptides, and/or proteins may further be linked, either covalently or non-covalently, to carbohydrate and/or lipid moieties. For example, they may be linked to mannan residues. The proteinaceous material comprises proline residues.

It has been surprisingly found that contacting a composition comprising mannoprotein with a prolyl-specific endoprotease may produce a wine stabiliser which when added to the wine in an effective amount may stabilize said wine by preventing or retarding the crystallisation of KHT and which may cause less formation of protein haze as compared to a composition comprising proteinaceous material which has not been contacted with a prolyl-specific endoprotease. A wine with no or little protein haze may be better appreciated by the customers than a wine with protein haze.

In the context of the invention "protein haze" shall refer to any precipitation, aggregation, and/or flocculation in wine, which said precipitation, aggregation, and/or flocculation comprises mannoprotein. In an embodiment preferably protein haze occurs through interaction of mannoprotein with polyphenols.

The wine stabiliser produced with the process according to the invention, when added to a wine may not react, or may react to a lesser extent, with polyphenols present in the wine, but may instead stay in solution, which may result in an increase of the efficacy of said stabiliser against crystallisation of KHT as compared to adding a stabiliser which causes protein haze.

The mannoprotein is preferably derived from yeast, preferably from Saccharomyces cerevisiae. Mannoprotein is defined herein according to Resolution Oeno 26/2004 of the OIV (Organisation Internationale de Ia Vigne et du Vin) as a product which can be extracted from Saccharomyces cerevisiae cells and/or yeast cell walls by physico-chemical or enzymatic methods. Mannoproteins are different structures depending on their molecular weight, their degree and type of glycosylation, and are known to be effective as a stabiliser. WO2008/128972 describes a wine stabiliser composition comprising at least 2.5% of a peptide mixture with a molecular weight between 3 kDa and 10 kDa, as determined by GPC as described in the materials and methods section of WO2008/128972.

The wine stabiliser produced by the process according to the invention may further comprise one or more biomolecules such as carbohydrates, lipids, fatty acids, and/or oligonucleotides.

Throughout the description of the invention "wine" may be understood to encompass wine before or after bottling as well as must at the end of the alcoholic fermentation, and wine during aging.

Any wine may be stabilised by the stabiliser produced by the process of the invention. Preferably the wine is aged on oak. In a preferred embodiment the wine is a red wine. Red wines are usually richer in tannins than white or rose wine, which tannins readily react with mannoprotein to form turbidity. However, also white or rose wines may be suitably stabilised, for example white or rose wines which are aged on oak, such as oak aged chardonnay.

The wine stabiliser produced according to the process of the invention may be effective in preventing or retarding the crystallisation of potassium salts of tartaric acid, or crystals of other organic acids which may be present in wine.

A "proline-specific protease" is defined herein as a protease which has a preference to cleave a peptide bond adjacent to a proline residue in a peptide chain, either at the carboxy-terminal side of the proline residue or at the amino-terminal side of the proline residue. The proline-specific protease is a proline-specific endoprotease. The proline-specific protease is a proline-specific endoprotease. Proline-specific endopeptidases include proline-specific oligopeptidases. Proline-specific oligopeptidases have a preference to cleave a peptide bond adjacent to a proline residue in peptide chains of about 30 amino acids or less, for example 30 amino acids or less. Examples of prolyl-oligopeptidases are prolyl- oligopeptidases belonging to the class EC 3.4.21.26. More preferably, the proline-specific endoprotease is a proline-specific endoprotease derived from fungi, more preferably from the genus Aspergillus, most preferably Aspergillus niger. The latter organism is widely known as a food grade microorganism. Most preferably the proline-specific endoprotease is a proline-specific endoprotease which cleaves at the carboxyterminal side of proline.

Examples of suitable fungal proline-specific endoproteases are those disclosed in WO02/45524. Surprisingly, we have found that the proline-specific endoproteases described in WO02/45524 may be used to treat compositions comprising proteinaceous material to produce a wine stabiliser which, when added to wine, may prevent or retard the crystallisation of KHT, but which may lead to no or little protein haze. Most preferred is the specific proline-specific endoprotease referred to in claims 1-5, 11 and 13 of WO02/45524.

The proline-specific endoprotease may be isolated via methods known in the art. For instance, proline-specific endoprotease may be isolated from culture broths obtained from (large scale) fermentation processes wherein the proline-specific endoprotease- producing microorganism, such as Aspergillus niger, is grown. In a preferred embodiment, the proline-specific endoprotease is isolated from a microbial host that is genetically engineered in order to over-express a gene encoding the proline-specific endoprotease. Suitable hosts known in the art are bacteria (i.e. of the genus Bacillus, Escherichia etceteras), yeasts (i.e. of the genus Saccharomyces or Kluyveromyes) or fungi (i.e. from the genus Aspergillus, such as Aspergillus niger, Aspergillus oryzea and others known in the art). Most preferred is the over-expression of the gene encoding an Aspergillus niger proline-specific endoprotease by an engineered Aspergillus niger host.

The specificity of a proline-specific endoprotease can be determined by methods that are generally known to the person skilled in the art. For example, the activity of proline- specific endoproteases that cleave at the carboxy-terminal side of proline, can be measured by using the synthetic peptide Z-Gly-Pro-pNA (Z = benzyloxycarbonyl) as a substrate while monitoring the formation of the yellow coloured para-nitroanilide (pNA). The activity of proline-specific endoproteases that cleave at the amino-terminal side of the proline residue can be identified using for example the synthetic substrate FA-Pro-Ala-Ala (FA = 3-(2- furylacryloyl)) and whereby the carboxy- terminus of the substrate preferentially is blocked by methods known in the art. The activity of proline-specific endoproteases that cleave in between two adjacent proline residues can be identified using for example an internally quenched fluorogenic substrate such as HOO-E-(EDANS)-PPPPK-(DABCYL)- NH2 according to the method described by Matayoshi et al (1990), Science 247, 954- 958. In this substrate, the fluorescent donor (EDANS) is linked to the side chain carboxyl group of the N-terminal glutamic acid and the fluorescent quenching acceptor (DABCYL) is linked to the side chain amino group of the C-terminal lysine.

As with any enzyme, the prolyl-specific endoprotease of the process of the invention requires water for its hydrolytic activity. Therefore, in a preferred embodiment, the composition comprising mannoprotein used in the process according to the invention is a liquid composition. A liquid composition is defined herein as a solution, preferably an aqueous solution.

In a preferred embodiment the temperature of the process of the invention may be between 5 and 120°C. More preferably the temperature is between 10 and 8O°C, more preferably between 20 and 75°C, even more preferably between 40 and 7O°C. At higher temperature enzymes reactions proceed faster. However, above certain temperatures, which are specific for any enzyme, enzymes tend to inactivate. Above 12O°C most known enzymes will inactivate. Below 5°C most enzymes are not active, or only to a small extent.

The pH of the process according to the invention may be anywhere between 2 and 10, preferably between 3 and 9. Most known prolyl-specific endoproteases are active around pH 8, such as from Flavobacterium (EP 0 967 285), Aeromonas (J.Biochem.1 13, 790-796), Xanthomonas, and Bacteroides. The prolyl-specific endoproteases from Aspergillus niger is active around pH 5 (EP 0 522 428).

The contacting of the process according to the invention is carried out for a time period ranging between 5 minutes and 1 week, more preferably between 30 minutes and 1 day.

The amount of prolyl-specific endoprotease in the process of the invention may vary between wide limits. In a preferred embodiment the amount of prolyl-specific endoprotease is between 0.001 and 1000 units of prolyl-specific endoprotease activity per gram mannoprotein, whereby the activity is determined by an activity measurements using Z-Gly-Pro-pNA as a substrate, more preferably between 0.01 and 100 units/gram mannoprotein, more preferably between 0,1 and 10 units / gram mannoprotein.

The amount of prolyl-specific endoprotease in the treatment is preferably such that a wine stabiliser is produced which, once added to the wine, may prevent or retard the crystallisation of KHT whilst causing no or little protein haze, and may depend on the temperature and pH of the composition comprising mannoprotein, as well as on the time period of the treatment of mannoprotein in the composition. For example, when the treatment is done at or near a temperature and/or pH at which the prolyl-specific endoprotease displays optimal enzyme activity (Tₒₚₜ and pHₒₚₜ) the amount of prolyl-specific endoprotease may be less in order to produce the desired stabiliser for wine of the invention. When the treatment is done at a temperature significantly below Tₒₚₜ, e.g. more than 10°C below Tₒₚₜ, and/or at a pH which is significantly lower or higher than the pHₒₚₜ, e.g. more than 1 pH unit away from pHₒₚₜ, the concentration of prolyl-specific endoprotease may be higher in order to produce the desired stabiliser for wine of the invention. Likewise, when the treatment time for a short period, and/or when the amount of the mannoprotein in the composition is high, the amount of prolyl-specific endoprotease may be higher in order to produce the wine stabiliser of the invention, whereas when the treatment time is for a longer period, and/or when the content of the mannoprotein in the composition is lower, the amount of prolyl-specific endoprotease may be lower in order to produce the wine stabiliser of the invention. The skilled person may therefore, without undue burden, establish suitable conditions with respect to the amount of prolyl-specific endoprotease in relation to the temperature and pH of the composition comprising mannoprotein, the time of treatment, and the content of the mannoprotein in the composition comprising proteinaceous material in order to produce the desired wine.

The process of the invention includes an inactivation step after the contacting step, for the purpose of inactivating the prolyl-specific endoprotease. The inactivation is done by heating, more preferably by boiling the composition of the process according to the invention. Boiling advantageously may cause precipitation of the (inactivated) prolyl-specific endoprotease. Optionally the process according to the invention may include a solid-liquid separation step after the contacting step, for example by centrifugation or by filtration. Preferably, the process according to the invention includes, after the contacting step and the inactivation step, a solid-liquid separation step. By inactivating the prolyl-specific endoprotease, the contacting of the active enzyme with the composition comprising the mannoprotein may be controlled such that a wine stabiliser is produced which, once added to the wine may prevent or retard the crystallisation of KHT whilst causing no or little protein haze. If the contacting of the active prolyl-specific endoprotease with the composition comprising the mannoprotein proceeds for too long, the desired wine stabiliser may not be obtained, for example because the mannoprotein has been cleaved to a too large an extent by the prolyl-specific endoprotease, which may lead to loss of the efficacy as a wine stabiliser. Removal of solids may result in a purer wine stabiliser and may avoid adding the (inactivated) prolyl- specific endoprotease to the wine which otherwise might be added concomitantly with the wine stabiliser to the wine.

Preferably the prolyl-specific endoprotease of the process according to the invention is an isolated or purified prolyl-specific endoprotease. An "isolated" or "purified" prolyl-specific endoprotease is defined herein as a prolyl-specific endoprotease which is isolated from its native environment. For example, recombinantly produced prolyl-specific endoproteases expressed in host cells are considered isolated for the purpose of the invention as are native or recombinant prolyl-specific endoproteases which have been purified to some extend by any suitable technique known in the art. The purified and/or isolated prolyl-specific endoprotease preferably contains little or no undesired contaminating components which might otherwise interfere with the composition comprising mannoprotein during the treatment. For example, the presence of proteases other than the prolyl-specific endoprotease together with the prolyl-specific endoprotease may break down the mannoprotein during the treatment, such that the efficacy of the stabiliser against the crystallisation of KHT may be diminished or lost.

### EXAMPLES

### MATERIAL AND METHODS

### Nucleation and crystal growth of KHT

The nucleation and crystal growth of KHT in wine can be measured and quantified by measuring the time of appearance of crystals in the wine when stored at minus 4°C. A visual inspection is performed daily and the time necessary to detect the appearance of crystals (Tcrys) is expressed in number of days (Moutounet et al. In : Actualites OEnologiques 1999 Vieme Symposium International d'Oenologie de Bordeaux (Lonvaud-Funel ed.)).

### Determination of prolyl-specific protease activity

The substrate solution is a 2 mM solution of N-carbobenzoxy-glycine-proline-p-nitro anilide (Z-Gly-Pro-pNA; molecular weight 426.43 ; Bachem) made in a 0.1 M citric acid / 0.2 M disodium phosphate buffer pH 5.0 containing 40 % dioxan.

To 1 ml. of buffer pH 5.0, 250 µl of the substrate solution is added followed by 100 µl of the enzyme solution (larger or smaller volume amounts of enzyme solution should be compensated for by buffer solution). The reaction mixture is incubated at 37°C and the release of pNA is followed by measuring the absorbance increase at 410 nm. Activity definition: 1 unit is the enzyme activity that liberates 1 µmol pNA from Z- Gly-Pro-pNA in 1 minute under described reaction conditions. In order to calculate concentrations a molar extinction coefficient (E) of 8,800 M<"1> is used.

### Prolyl-specific endoprotease

Prolyl-specific endoprotease from Aspergillus niger G306 (deposited at the Centraal Bureau voor Schimmelcultures CBS (CBS109712) on 10 September 2001) was produced as described in WO02/45524.

### Mannoprotein

Mannoproteins were recovered from yeast extract (Maxarome®, DSM Food Specialties) by washing the yeast extract approximately 5-fold with water by means of ultrafiltration (UF) over a 4 kDa Nadir® polyethersulfone hydrophilic membrane (Microdyn-Nadir, Germany) until a concentration of 10% w/v). This is "the mannoprotein solution".

### LC/MS analysis of mannoprotein

UHPLC (ultra high performance liquid chromatography) using an ion trap mass spectrometer (LTQ) (Thermofishert™, Breda, the Netherlands) coupled to an Accela pump (Thermofisher™, Breda, the Netherlands) was used in characterizing the mannoprotein samples produced by the inventive enzyme mixture. The peptides formed were separated using an Agilent SB-C18, 2.1*5 mm (827700-902, Agilent) column in combination with a gradient of 0.1% formic acid in Milli Q water (Millipore, Bedford, MA, USA; Solution A) and 0.1 % formic acid in acetonitrile (Solution B) for elution. The gradient started at 100% of Solution A and increased to 30% of solution B in 45 minutes and was kept at the latter ratio for another 5 minutes. The injection volume used was 50 microliters, the flow rate was 400 microliter per minute and the column temperature was
maintained at 30°C. The total peptide concentration of the injected samples was approx. 1 mg/milliliter.

Detailed information on the individual peptides was obtained by using the "scan dependent" MS/MS algorithm which is a characteristic algorithm for an ion trap mass spectrometer.

Full scan analysis was followed by zoom scan analysis for the determination of the charge state of the most intense ion in the full scan mass range. Subsequent MS/MS analysis of the latter ion resulted in partial peptide sequence information, which could be used for database searching using the Sorcerer 2 (SageN, Milpitas, CA, USA). Data were searched against the Saccharomyces cerevisiae database (SGD). The reliability of the database searching technique was increased by omitting those MS/MS spectra with a probability higher then 0.9.

### Determination of the molar fraction of peptides (%) of Saccharomyces cerevisiae mannoprotein, carrying a carboxyterminal proline

LC/MS/MS can be used for the analysis of the C-terminus of a peptide. With an algorithm in which the peptide's molecular mass (analyzed with LC/MS) and its (partial) amino acid sequence (analyzed with LC/MS/MS) are linked with automatic search procedures within protein databanks, complex peptide mixtures can be analyzed. These options have enabled us to quantify the incidence of peptides carrying a carboxy terminal proline residue. Owing to the limitations set by the Agilent SB-C18 column used, only peptides with a molecular weight between roughly 400 and 2000 Dalton are analysed using this technique. Fortunately, in the mannoprotein samples a majority of the peptides have such molecular weights.

To determine the molar fraction of peptides carrying a carboxyterminal proline in the mannoprotein samples before and after incubation with prolyl-specific protease, individual peptide peaks eluting from the Agilent SB-C18 column are selected and partial carboxyterminal amino acid sequences are determined using the techniques specified above. Analysis of at least 100, preferably at least 150 and more preferably between 200 to 300, for example 150 of the most abundant, randomly chosen peptides thus provides insight in the frequency in which peptides carrying a proline residue at the carboxyterminus of the peptide occur. The quotient of the number of peptides found to carry a carboxyterminal proline residue times 100 and the total number of peptides analysed thus provides the molar fraction of peptides (%) carrying a carboxyterminal proline.

### Example 1

### Incubation with prolyl-specific protease

To the mannoprotein solution prolyl-specific endoprotease was added such that the concentration was 1 unit / gram mannoprotein.

The pH of the mannoprotein solution was not changed (pH 5.3). The mannoprotein solution was incubated at 50°C. Samples of 1 ml. were drawn at different incubation times. To the remaining 5 ml. mannoprotein solution prolyl-specific endoprotease was added such that the concentration was 3 units / gram mannoprotein solution, after which a sample was drawn at t=24h and t=41 h.

All samples (1 ml.) were heated for 1.5 minutes in boiling water and diluted with 9 ml of water. The solution was centrifuged in order to remove any denatured prolyl-specific endoprotease. The samples with double dosage of enzyme had three times as much pellet, which confirmed that the boiling and centrifugation step actually removed the enzyme.

### Testing the formation of protein haze (turbidity)

Mannoprotein which was treated with prolyl-specific endoprotease was added to a young red wine (Veneto, It) and to a barrel-aged red wine (Gran Feudo reserva, Julian Chivite, Navarra, 2001) in a concentration of 200 mg/L (on dry matter basis). Samples of 25 ml. wine were placed at +4°C, and the protein haze was measured as the turbidity by means of a HACH 2100AN turbimeter. Results are shown in Table 1 and 2.

### Results

**Table 1: Turbidity (NTU) of red wine (Veneto) comprising mannoprotein (200 mg/L wine), which mannoprotein has been treated with prolyl-specific endoprotease (1 unit/gram mannoprotein). Turbidity values are expressed as a function of incubation time of the mannoprotein with said prolyl-specific endoprotease, and as a function of storage time of said red wine at 4°C after addin the prol l-specific endoprotease treated mannoprotein.**

| Storage time of the wine (days) after addition of the prolyl-specific endoprotease treated mannoprotein to the wine | **1 day** | **3 days** | **7 days** |
|---|---|---|---|
| Incubation time of the mannoprotein with prolyl-specific endoprotease (hours) | | | |
| 0 hours, comparative example | 21.8 | 27.4 | 44 |
| 2 hours | 10.0 | 16.1 | 30.1 |
| 4 hours | 7.8 | 13.3 | 26.2 |
| 7 hours | 6.3 | 11.5 | 23.2 |
| 16 hours | 4.3 | 8.2 | 18 |
| 23 hours | 3.9 | 7.4 | 16.6 |
| 24 hours d* | 2.8 | 5.8 | 13.8 |
| 41 hours d* | 2.3 | 5.1 | 13 |
| Wine without added mannoprotein | 0.3 | 0.4 | 1.6 |

| | | | |
|---|---|---|---|
| *3 units / gram mannoprotein | | | |

The efficacy of the prolyl-specific endoprotease incubated mannoprotein was measured in Listel rose and French Chardonnay (Tables 3 and 4).

**Table 2: Turbidity (NTU) of Gran Feudo reserva, Julian Chivite, Navarra, 2001 comprising mannoprotein (200 mg/L wine), which mannoprotein has been treated with prolyl-specific endoprotease (1 unit/gram mannoprotein). Turbidity values are expressed as a function of incubation time of the mannoprotein with said prolyl-specific endoprotease, and as a function of storage time of said red wine at 4°C after adding the prolyl-specific endoprotease treated mannoprotein.**

| Storage time of the wine (days) after addition of the prolyl-specific endoprotease treated mannoprotein to the wine | **1 day** | **4 days** | **7 days** |
|---|---|---|---|
| Incubation time of the mannoprotein with prolyl-specific endoprotease (hours) | | | |
| 0 hours, comparative example | 11.5 | 38.5 | 43.3 |
| 2 hours | 8.2 | 32 | 40 |
| 4 hours | 6.6 | 28.7 | 37.2 |
| 7 hours | 5.7 | 18.5 | 21.8 |
| 16 hours | 4.1 | 13.7 | 18.6 |
| 23 hours | 3.5 | 13 | 18.6 |
| Blank wine* | 0.255 | 0.38 | 3.9 |

| | | | |
|---|---|---|---|
| *wine stored at +4°C | | | |

**Table 3: Efficacy of mannoprotein as a wine stabiliser after treatment of the mannoprotein with 1 unit prolyl-specific endoprotease per gram mannoprotein, at different incubation times of the mannoprotein (hours) with prolyl-specific endoprotease, measured in rose wine, expressed as the number of days (d) of the first appearance of crystals (Tcrys) after storage of the wine at minus 4°C.**

| incubation time of the mannoprotein with prolyl-specific endoprotease (hours) | 0h | 2h | 4h | 7h | 16h | 23h | 24h 3 units / gram mannoproteins | 41h 3 units / gram mannoproteins |
|---|---|---|---|---|---|---|---|---|
| concentration mannoprotein in the wine (mg/L) | | | | | | | | |
| + 0 mg/l | 1d | 1d | 1d | 1d | 1d | 1d | 1d | 1d |
| +50mg/l | <3d | <3d | <3d | <3d | <3d | <3d | <3d | <3d |
| +100 mg/l | <3d | <3d | <3d | <3d | <3d | <3d | <3d | <3d |
| +200 mg/l | 6d | 6d | 7d | 7d | 7d | 4d | 3d | 3d |

**Table 4: Efficacy of mannoprotein as a wine stabiliser after treatment of the mannoprotein with 1 unit prolyl-specific endoprotease per gram mannoprotein, at different incubation times of the mannoprotein (hours) with prolyl-specific endoprotease, measured in Chardonnay, expressed as the number of days (d) of the first appearance of crystals (Tcrys) after storage of the wine at minus 4°C.**

| incubation time of the mannoprotein with prolyl-specific endoprotease (hours) | No incubatio n with prolyl-specific endo protease | 0h | 2h | 4h | 7h | 16h | 23h | 24h (3 units/ gram manno proteins) | 41h (3 units/ gram manno proteins) |
|---|---|---|---|---|---|---|---|---|---|
| concentration mannoproteins in the wine (mg/L) | | | | | | | | | |
| 0 mg/l | 1d | 1d | 1d | 1d | 1d | 1d | 1d | 1d | 1d |
| 50mg/l | 3d | 3d | 3d | 3d | 3d | 3d | 3d | 3d | 3d |
| 100 mg/l | 5d | 5d | 5d | 5d | 5d | 6d | 6d | 6d | 6d |
| 150 mg/l | 10d | 10d | 10d | 12d | 10d | 10d | 10d | 10d | 7d |
| 200 mg/l | 15d | 11d | 14d | 14d | 12d | 13d | 13d | 11d | 11d |

### Example 2

### Incubation with prolyl-specific protease

To the mannoprotein solution isolated prolyl-specific endoprotease was added such that the concentration was 1 unit / gram mannoprotein.

The pH of the mannoprotein solution was not changed (pH 5.3). The mannoprotein solution was incubated at 5O°C. Samples of 1 ml. were drawn at t=0h, t=1 h, t=2h, t=4h, t=6h, t=9h, t=25h, and t=48h.

All samples (1 mlL) were heated for 1.5 minutes in boiling water and diluted with 16.5 mL of water. The solution was centrifuged in order to remove any denatured prolyl-specific endoprotease.

### Testing the formation of protein haze (turbidity)

Mannoprotein which was treated with isolated prolyl-specific endoprotease was added to a barrel-aged red wine (Gran Feudo reserva, Julian Chivite, Navarra, 2001) in a concentration of 200 mg/L (on dry matter basis). Samples of 25 ml. wine were placed at +4°C, and the protein haze was measured after 1, 4, and 7 days as the turbidity by means of a HACH 2100AN turbimeter. Results are shown in Table 5.

### Results

**Table 5: Turbidity (NTU) red wine comprising mannoprotein (200 mg/L wine), which mannoprotein has been treated with prolyl-specific endoprotease (1 unit/gram mannoprotein). Turbidity values are expressed as a function of incubation time of the mannoprotein with said prolyl-specific endoprotease, and as a function of storage time of said red wine at 4°C after adding the prolyl-specific endoprotease treated mannoprotein.**

| Storage time of the wine (days) after addition of the prolyl-specific endoprotease treated mannoprotein to the wine | **1 day** | **4 days** | **7 days** |
|---|---|---|---|
| Incubation time of the mannoprotein with prolyl-specific endoprotease (hours) | | | |
| 0 hours (no prolyl-specific endoprotease added) | 20.1 | 41 | 54.5 |
| 0 hours | 17.0 | 39.6 | 47 |
| 1 hours | 6.9 | 32.1 | 40.1 |
| 2 hours | 6.6 | 26.7 | 36.2 |
| 4 hours | 5.2 | 21 | 29.3 |
| 6 hours | 4.6 | 17.7 | 19.9 |
| 9 hours | 4.7 | 13.2 | 16.2 |
| 25 hours | 3.8 | 6.99 | 16 |
| 48 | 3.8 | 6.48 | 13.2 |
| Blank wine* | 0.255 | 0.38 | 3.9 |

| | | | |
|---|---|---|---|
| *wine stored at +4°C | | | |

The efficacy of the isolated prolyl-specific endoprotease incubated mannoprotein was measured in fresh French Chardonnay. First, part of the wine was cold-stabilised by storage at minus 4°C whilst the remainder wine was untreated. Next, two wine mixtures were prepared comprising the untreated wine and the cold-stabilised wine: wine mixture A was prepared by mixing 80 parts of the untreated wine with 20 parts of the cold- stabilised wine; mixture B was prepared by mixing 50 parts of the untreated wine with 50 parts of the cold-stabilised wine. Results shown in Tables 6 and 7.

**Table 6: Efficacy of mannoprotein as a wine stabiliser after treatment of the mannoprotein with 1 unit prolyl-specific endoprotease per gram mannoprotein, at different incubation times of the mannoprotein (hours) with isolated prolyl-specific endoprotease, measured in wine mixture A, expressed as the number of days (d) of the first appearance of crystals (Tcrys) after storage of the wine at minus 4°C.**

| incubation time of the mannoprotein with isolated prolyl-s pecific endoprotease (hours) | * | 0h | 1h | 2h | 4h | 6h | 9h | 25h | 28h | 31h | 48h | 57h | 72h |
|---|---|---|---|---|---|---|---|---|---|---|---|---|---|
| concentration mannoprotein in the wine (mg/L) | | | | | | | | | | | | | |
| 50 mg/L | 3d | 3d | 3d | 3d | 3d | 3d | 3d | 3d | 3d | 3d | 3d | 3d | 3d |
| 100 mg/L | 3d | 3d | 3d | 3d | 3d | 3d | 3d | 3d | 3d | 3d | 3d | 3d | 3d |
| 200 mg/L | 12d | 10d | 11d | 11d | 10d | 11d | 10d | 9d | 9d | 12d | 11d | 11d | 10d |

| | | | | | | | | | | | | | |
|---|---|---|---|---|---|---|---|---|---|---|---|---|---|
| *no prolyl-specific endoprotease added | | | | | | | | | | | | | |

**Table 7: Efficacy of mannoprotein as a wine stabiliser after treatment of the mannoprotein with 1 unit prolyl-specific endoprotease per gram mannoprotein, at different incubation times of the mannoprotein (t, in hours) with isolated prolyl-specific endoprotease, measured in wine mixture B, expressed as the number of days (d) of the first appearance of crystals (Tcrys) after storage of the wine at minus 4°C.**

| incubation time of the mannoprotein with isolated prolyl-specific endoprotease (hours) | * | 0h | 1h | 2h | 4h | 6h | 9h | 25h | 28h | 31h | 48h | 57h | 72h |
|---|---|---|---|---|---|---|---|---|---|---|---|---|---|
| concentration mannoprotein in the wine (mg/L) | | | | | | | | | | | | | |
| 50 mg/L | 10d | 6d | 4d | 4d | 5d | 4d | 3d | 3d | 4d | 3d | 3d | 3d | 5d |
| 100 mq/L | 13d | 13d | 12d | 14d | 10d | 14 | 11d | 14d | 6d | 6d | 14d | 11d | 6d |
| 200 mg/L | 40d | 38d | 38d | 40d | 18d | 40d | 40d | 24d | 24d | 28d | 21d | 24d | 28d |

| | | | | | | | | | | | | | |
|---|---|---|---|---|---|---|---|---|---|---|---|---|---|
| *no prolyl-specific endoprotease added | | | | | | | | | | | | | |

### Example 3

### Molar incidence of peptides carrying proline as the carboxy terminal residue

The mannoprotein solutions of Example 2 which were incubated with prolyl-specific endoprotease for 4 hours (Sample 1) and 25 hours (Sample 2), as well as the non-incubated mannoprotein solution (Sample 0) were analyzed by LC-MS/MS for the incidence of peptides carrying a carboxy-terminal prolyl residue. Results are shown in Table 8.

**Table 8: Molar incidence of yeast mannoprotein peptides carrying proline as the carboxy terminal residue.**

| **Sample** | **Number of peptides with Proline at C-terminus** | **Total number of peptides determined** | **Ratio (%)** |
|---|---|---|---|
| 0 | 0 | 434 | 0 |
| 1 | 24 | 333 | 7 |
| 2 | 17 | 249 | 7 |

## Claims

1. Process to produce a wine stabiliser comprising:
(a) contacting a composition comprising a mannoprotein with a prolyl-specific endoprotease for a period of between 5 minutes and 1 week, and
(b) inactivating the prolyl-specific endoprotease of step (a) by heating the prolyl-specific endoprotease to produce a wine stabiliser capable of reducing or preventing the nucleation and/or the growth of crystals of potassium hydrogen tartrate (KHT) and of reducing or preventing a protein haze in wine when the wine stabiliser is added to a wine.

2. Process according to claim 1 wherein the temperature is between 5 and 120°C.

3. Process according to claim 1 or 2 wherein the pH is between 2 and 10.

4. Process according to any one of claims 1-3 wherein the amount of prolyl-specific endoprotease is between 0.001 and 1000 units of prolyl-specific endoprotease activity per gram mannoprotein, whereby the activity is determined by an activity measurements using Z-Gly-Pro-pNA as a substrate.

5. Process according to any one of claims 1-4 including after the inactivation step undertaking a solid-liquid separation step.

6. Process according to any one of claims 1- 5 wherein the prolyl-specific endoprotease is an isolated or purified prolyl-specific endoprotease.

## Patentansprüche

1. Verfahren zur Herstellung eines Wein-Stabilisators, umfassend:
(a) in Kontakt bringen einer Zusammensetzung, umfassend ein Mannoprotein, mit einer prolyl-spezifischen Endoprotease für einen Zeitraum zwischen 5 Minuten und einer Woche, und
(b) Inaktivieren der prolyl-spezifischen Endoprotease aus Schritt (a) durch Erhitzen der prolyl-spezifischen Endoprotease zur Herstellung eines Wein-Stabilisators, der die Keimbildung und/oder das Wachstum von Kaliumhydrogentartrat (KHT)-Kristallen reduzieren oder verhindern kann und der eine Eiweißtrübung in Wein reduzieren oder verhindern kann, wenn der Wein-Stabilisator dem Wein hinzugefügt wird.

2. Verfahren nach Anspruch 1, bei dem die Temperatur zwischen 5 und 120°C beträgt.

3. Verfahren nach Anspruch 1 oder 2, bei dem der pH-Wert zwischen 2 und 10 liegt.

4. Verfahren nach einem der Ansprüche 1 bis 3, bei dem die Menge prolylspezifischer Endoprotease zwischen 0,001 und 1000 Einheiten prolylspezifischer Endoprotease-Aktivität pro Gramm Mannoprotein beträgt, wobei die Aktivität bestimmt wird durch eine Aktivitätsmessung, welche Z-Gly-Pro-pNA als ein Substrat verwendet.

5. Verfahren nach einem der Ansprüche 1 bis 4, umfassend nach dem Inaktivierungs-Schritt einen Feststoff-Flüssigkeits-Trennungsschritt.

6. Verfahren nach einem der Ansprüche 1 bis 5, bei dem die prolyl-spezifische Endoprotease eine isolierte oder gereinigte prolyl-spezifische Endoprotease ist.

## Revendications

1. Procédé pour produire un stabilisant de vin comprenant le fait :
(a) de mettre en contact une composition comprenant une mannoprotéine avec une endoprotéase spécifique de prolyle pendant une période comprise entre 5 minutes et 1 semaine, et
(b) d'inactiver l'endoprotéase spécifique de prolyle de l'étape (a) en chauffant l'endoprotéase spécifique de prolyle pour produire un stabilisant de vin capable de réduire ou d'empêcher la nucléation et/ou la croissance de cristaux de bitartrate de potassium (KHT) et de réduire ou d'empêcher un trouble protéique dans le vin lorsque le stabilisant de vin est ajouté au vin.

2. Procédé selon la revendication 1, dans lequel la température est comprise entre 5 et 120°C.

3. Procédé selon la revendication 1 ou 2, dans lequel le pH est compris entre 2 et 10.

4. Procédé selon l'une quelconque des revendications 1 à 3, dans lequel la quantité d'endoprotéase spécifique de prolyle est comprise entre 0,001 et 1000 unité(s) d'activité endoprotéase spécifique de prolyle par gramme de mannoprotéine, moyennant quoi l'activité est déterminée par des mesures d'activité en utilisant Z-Gly-Pro-pNA comme substrat.

5. Procédé selon l'une quelconque des revendications 1 à 4 comportant, après l'étape d'inactivation, le fait de procéder à une étape de séparation solide-liquide.

6. Procédé selon l'une quelconque des revendications 1 à 5, dans lequel l'endoprotéase spécifique de prolyle est une endoprotéase spécifique de prolyle isolée ou purifiée.
